(19) Europäisches Patentamt
European Patent Office
Office européen des brevets

(11) Numéro de publication: **0 056 210**
**B1**

(12) **FASCICULE DE BREVET EUROPEEN**

(45) Date de publication du fascicule du brevet: **12.03.86**

(51) Int. Cl.[4]: **C 07 K 5/08**, C 07 K 5/10, C 12 Q 1/34, G 01 N 33/52

(21) Numéro de dépôt: **81402087.1**

(22) Date de dépôt: **29.12.81**

(54) **Procédé pour le dosage fluorimétrique des endotoxines, nouveaux peptides portant un fluorophore utilisables dans ledit procédé et leur méthode de préparation.**

(30) Priorité: **09.01.81 FR 8100261**

(43) Date de publication de la demande: **21.07.82 Bulletin 82/29**

(45) Mention de la délivrance du brevet: **12.03.86 Bulletin 86/11**

(84) Etats contractants désignés: **AT BE CH DE FR GB IT LI LU NL SE**

(56) Documents cités:
**EP - A - 0 000 063**
**EP - A - 0 004 256**
**EP - A - 0 018 112**

**CHEMICAL ABSTRACTS, vol. 91, no. 19, 5 novembre 1979, page 179, abrégé 152573f COLUMBUS OHIO (US)**

(73) Titulaire: **CENTRE NATIONAL DE LA RECHERCHE SCIENTIFIQUE (CNRS), 15, Quai Anatole France, F-75700 Paris (FR)**

(72) Inventeur: **Monsigny, Michel, 341, rue des Bouvreuils, F-45590 Saint-Cyr-en-Val (FR)**

(74) Mandataire: **Nony, Michel et al, Cabinet Nony 29, rue Cambacérès, F-75008 Paris (FR)**

**EP 0 056 210 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé pour le dosage fluorimétrique des endotoxines (lipopolysaccharides ou lipopolyosides bactériens) mettant en œuvre un peptide portant un fluorophore. L'invention concerne également de nouveaux peptides portant un fluorophore utilisables dans ledit procédé et leur méthode de préparation.

Il est connu de doser les endotoxines par mesure de l'élévation de température provoquée chez le lapin (Pharmacopée française, IXe édition, II-235 à II-238); par mesure du temps de formation d'un gel à partir de lysat d'amébocyte du Limule (Cooper et al., J. Lab. Cli. Med., July, 1971, 138–148); par colorimétrie soit à l'aide de peptidyl-p-nitroanilide (Iwanaga et al., Haemostasis 7, 183–188, 1978; Harada et al., Progress in Clinical and Biological research, vol. 29, 1979, 209–220), soit à l'aide de peptidyl-naphtylamine, peptidyl-nitronaphtylamine, peptidyl-méthoxynaphtylamine, peptidyl-aminoquinoléine, peptidyl-aminonitroquinoléine (demande de brevet européen 41 089); par fluorimétrie à l'aide de peptidyl-AMC (brevet JP-A-79 68 290). Ces méthodes sont une sensibilité insuffisante pour permettre de détecter de très faibles quantités d'endotoxines (quantités de l'ordre du picogramme).

Il a également été proposé (cf. le brevet des Etats-Unis d'Amérique n° 4 188 264) de déterminer les endotoxines par une méthode fluorimétrique mettant en œuvre un substrat peptidique de formule:

R–Gly–Arg–R′ (A)

dans laquelle R est un reste d'acide aminé L ou de peptide, dont le groupe amino terminal est protégé par un groupe protecteur, et R′ est le reste de la β-naphtylamine ou d'un composé hydroxylé fluorescent choisi parmi les α- et β-naphtol, l'indoxyle, le N-méthyl indoxyle, la méthyl-4 umbelliférone et la résorufine. Les substrats peptidiques de formule (A) pour lesquels R′ représente le reste d'un composé hydroxylé fluorescent présentent l'inconvénient de s'hydrolyser spontanément en solution aqueuse.

Il a maintenant été trouvé, conformément à la présente invention, un procédé de dosage fluorimétrique des endotoxines dans les liquides qui permet de déterminer quantitativement les endotoxines avec une sensibilité très élevée. Ce procédé ne présente pas l'inconvénient signalé cidessus pour la méthode fluorimétrique mettant en œuvre les substrats peptidiques de formule (A) pour lesquels R′ est le reste d'un composé hydroxylé fluorescent. En outre il présente une sensibilité nettement supérieure à celle de la méthode fluorimétrique mettant en œuvre les substrats peptidiques de formule (A) pour lesquels R′ est le reste de la β-naphtlyamine.

Le procédé selon l'invention consiste à mettre en contact, à une température située dans l'intervalle 25°C-37°C, le liquide à analyser avec un lysat d'amébocytes du limule et une solution aqueuse d'un peptide de formule:

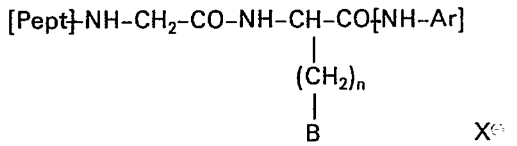
(I)

dans laquelle [Pept}- désigne une chaîne peptidique hydrophobe, n est égal à 1, 2, 3, 4 ou 5, B est un radical

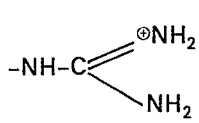
ou
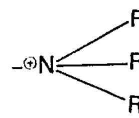
, $R_1$, $R_2$ et $R_3$ désignant des atomes d'hydrogène ou des groupes méthyle, {NH-Ar] désigne le reste de l'amino-3 éthyl-9 carbazole ou de l'amino-7 nitro-4 oxa-2 benzodiazole-1,3 et $X^{\ominus}$ désigne un anion, à régler si nécessaire le pH du milieu ainsi obtenu à une valuer comprise dans l'intervalle 6 à 7,5, et à doser par fluorimétrie l'amine hétéroaromatique $Ar–NH_2$ formée.

L'amine hétéroaromatique fluorescente $Ar–NH_2$ a un spectre de fluorescence fortement déplacé par acylation de l'amine. L'intensité de fluorescence à la longueur d'onde d'émission de ladite amine libre est au moins 100 fois plus grande que l'intensité de fluorescence, à cette même longueur d'onde, des composés de formule (I) correspondants. L'amine hétéromatique fluorescente $Ar–NH_2$ est soit l'amino-7 nitro-4 oxa-2 benzodiazole-1,3, soit l'amino-3 éthyl-9 carbazole.

Dans la formule (I) ci-dessus, «n» est de préférence égal à 3 et

B est de préference un groupe 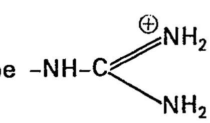

Le nombre des chaînons acides aminés de la chaîne peptidique [Pept}- des peptides de formule (I) n'est pas limité. Ce nombre est toutefois égal de préférence à 1 ou 2. Comme exemples de chaîne peptidique hydrophobe [Pept}- on peut citer les chaînes suivantes:

[Val-Leu}-
[Ser (OBz)}-
[Ile-Glu (γ-$OCH_3$)}-
[Val}-
[Leu}-
[Val-Ser}-

chaînes dans lesquelles Val désigne le chaînon valine, Leu le chaînon leucine, Ile le chaînon isoleucine, Ser le chaînon sérine, Ser (OBz) le chaînon sérine dans lequel le groupe $CH_2OH$ est remplacé par le groupe $CH_2–O–CH_2–C_6H_5$, et Glu (γ-$OCH_3$) le chaînon acide glutamique dans lequel le groupe COOH en position γ est remplacé par le groupe $COOCH_3$.

Dans les exemples de chaîne peptidique hydrophobe [Pept}- ci-dessus, le groupe amino $NH_2$ du chaînon acide aminé, lorsqu'il n'y a qu'un seul chaînon, et le groupe amino $NH_2$ du chaînon acide aminé de gauche, lorsqu'il y a deux chaînons, peuvent être non substitués ou substitués par un groupe tertiobutyloxycarbonyle (t Boc), benzyloxycarbonyle, benzyle, tosyle ou acyle, en particulier acétyle ou benzoyle.

Le procédé de dosage des endotoxines selon l'invention est basé sur la propriété que possèdent les endotoxines de rendre active une protéase contenue dans le lysat d'amébocytes du limule (Lumulus polyphemus). En l'absence d'endotoxine, la protéase n'agit pas sur les peptides de formule (I). En présence d'endotoxine, la protéase réalise l'hydrolyse des peptides de formule (I) avec libération d'une part des peptides de formule:

[Pept}-NH-$CH_2$-CO-NH-CH-COOH
|
$(CH_2)_n$ $X^{\ominus}$ (II)
|
B

dans laquelle [Pept}-, n, B et $X^{\ominus}$ ont les mêmes significations que dans la formule (I), et d'autre part de l'amine hétéroaromatique fluorescente Ar-$NH_2$. L'activité de la protéase est strictement proportionnelle à la concentration en endotoxine du milieu. Par suite la concentration du milieu en amine Ar-$NH_2$ libérée et l'intensité de fluorescence mesurée à la longueur d'onde d'émission de ladite amine libre sont, à un moment déterminé après la mise en contact du liquide contenant l'endotoxine, du lysat d'amébocytes du limule et du peptide de formule (I), strictement proportionnelles à la concentration en endotoxine du milieu.

Selon un premier mode de réalisation du procédé selon l'invention, le liquide à analyser, le lysat d'amébocytes du limule et la solution aqueuse du peptide de formule (I) sont laissés en contact, après réglage éventuel du pH et à la température indiquée précédemment, pendant un temps déterminé fixé à l'avance, qui est de préférence de 10 minutes à 30 minutes, puis on ajoute au milieu un composé qui arrête l'hydrolyse du peptide de formule (I) sans gêner la fluorescence de l'amine hétéroaromatique Ar-$NH_2$ formée, et on dose l'amine hétéroaromatique Ar-$NH_2$ formée en mesurant l'intensité de fluorescence à la longueur d'onde d'émission de l'amine libre. Comme composés arrêtant l'hydrolyse du peptide de formule (I) sans gêner la fluorescence de l'amine hétéroaromatique Ar-$NH_2$ formée on peut citer la benzamidine, l'ortho-amino benzamidine et la para-amino benzamidine.

Selon un deuxième mode de réalisation du procédé selon l'invention, l'intensité de fluorescence à la longueur d'onde d'émission de l'amine libre est enregistrée en continu dès la mise en contact du liquide à analyser, du lysat d'amébocytes du limule et de la solution aqueuse du peptide de formule (I) et le réglage éventuel du pH, la température étant maintenue constante à une valeur située dans l'intervalle défini précédemment. Cette deuxième façon d'opérer, dite méthode cinétique, permet d'ajuster sans tâtonnements la durée de l'hydrolyse à la quantité d'endotoxines présente dans l'échantillon à analyser.

Le procédé selon l'invention permet de doser les endotoxines dans des liquides divers (solutions aqueuses injectables de produits naturels ou de synthèse, solutions de protéines, liquides physiologiques tels que sérum, plasma, urine, etc.). Il est particulièrement sensible puisqu'il permet de détecter des quantités d'endotoxine de l'ordre du centième de picogramme. De plus il permet une détermination quantitative des endotoxines avec une précision de l'ordre de quelques pour cent, il est rapide et peut être rendu automatique. A titre de comparaison, la méthode basée sur la mesure du temps de formation d'un gel ne permet pas de détecter des quantités d'endotoxine inférieures à 30 picogrammes, est relativement longue (durée: 1 heure) et n'est que semi-quantitative.

Les peptides de formule (I) pour lesquels -{NH-Ar] est le reste de l'amino-7 nitro-4 oxa-2 benzodiazole-1,3 ou de l'amino-3 éthyl-9 carbazole sont des produits nouveaux et font partie en tant que tels de l'invention.

De façon générale, ces peptides de formule générale (I) peuvent être préparés par action, en présence d'un composé activateur du groupe COOH et d'hydroxy-1 benzotriazole, de l'amine hétéroaromatique fluorescente Ar-$NH_2$ sur les peptides de formule générale:

[Pept}-NH-$CH_2$-CO-NH-CH-COOH
|
$(CH_2)_n$ $X^{\ominus}$ (II)
|
B

dans laquelle [Pept}-, n, B et $X^{\ominus}$ ont les mêmes significations que dans la formule (I). Comme composé activateurs du groupe COOH utilisables, on peut citer ceux mentionnés par Konig W., Geiger R., Chem. Ber., 103, (1970), 788–798 et par Gross E., Meienhofer J. (The peptides: Analysis, synthesis and biology. I. Major methods of peptide bond formation, Academic Press, 1979, p. 435), et en particulier le dicyclohexylcarbodiimide.

Les peptides de formule générale (II) sont des produits connus qui peuvent être obtenus par les procédés classiques de synthèse peptidique (cf. Konig W., Geiger R. et Gross E., Meienhofer J., déjà cités).

Les exemples suivants illustrent l'invention sans la limiter.

Exemple 1

Préparation des peptides de formule (I)

Une millimole du peptide de formule:

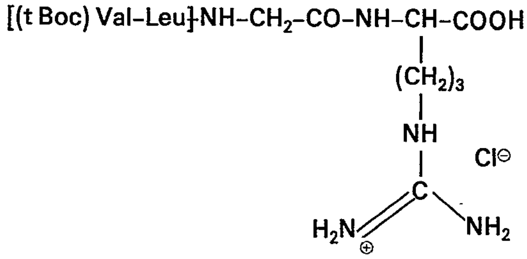

(III)

dans laquelle Leu désigne le chaînon leucine et (t Boc) Val le chaînon valine dans lequel le groupe amino $NH_2$ est substitué par un groupe tertiobutyloxycarbonyle, et une millimole d'acide p-toluènesulfonique sont dissoutes dans 2 ml de diméthylformamide redistillé dépourvu d'amines. A cette solution, refroidie à 0°C et maintenue sous atmosphère d'azote, on ajoute une millimole d'amino-3 éthyl-9 carbazole, une millimole d'hydroxy-1 benzotriazole et une millimole de dicyclohexylcarbodiimide. Le mélange réactionnel est maintenu 2 heures à 0°C, puis 18 heures à 25°C. La dicyclohexylurée formée est précipitée en maintenant le mélange à 0°C pendant 24 heures, puis est séparée par filtration ou centrifugation. Le diméthylformamide est éliminé par évaporation sous pression déduite. Le résidu obtenu, qui contient, à l'état brut, le peptide portant le fluorophore, est dissous dans un mélange contenant 80 parties en volume de chloroforme, 20 parties en volume de méthanol et 3 parties en volume d'eau, puis est fixé sur une colonne de gel de silice, et on élue avec le mélange ci-dessus. Les fractions contenant le peptide portant le fluorophore sont évaporées sous pression réduite.

On obtient ainsi, à l'état pur, le peptide de formule:

(IV)

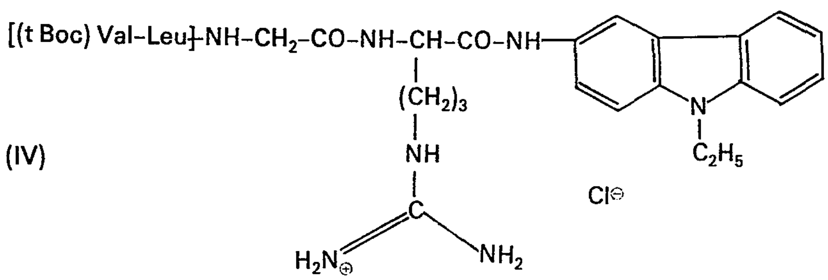

La pureté et l'identité du produit obtenu sont prouvées par, entre autres, l'analyse par chromatographie en couche mince et les spectres d'absorption et de fluorescence. Contrairement à l'amino-3 éthyl-9 carbazole qui, lorsqu'il est excité à 362 nm, présente une fluorescence très intense (200 unités de fluorescence) à 460 nm, le peptide de formule (IV), lorsqu'il est excité à 362 nm, ne présente qu'une fluorescence très faible (1 unité de fluorescence) à 460 nm.

Exemple 2

Préparation d'un peptide de formule (I) dérivé de l'amino-7 nitro-4 oxa-2 benzodiazole-1,3

En opérant comme dans l'exemple 1, mais en remplaçant l'amino-3 éthyl-9 carbazole par l'amino-7 nitro-4 oxa-2 benzodiazole-1,3, on obtient le peptide de formule (VII) ci-après.

(VII)

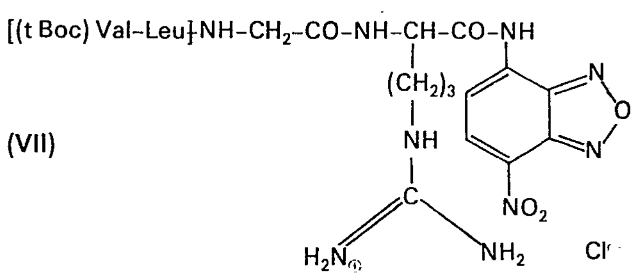

Exemple 3

Application des peptides de formule (I) au dosage des endotoxines

On dissout dans l'eau une quantité de peptide de formule (IV) telle que la solution obtenue présente, en cuve d'épaisseur 1 cm et à la longueur d'onde de 340 nm, une absorption égale à 0,1. A 400 µl de cette solution on ajoute 200 µl de lysat d'amébocytes de limule (lysat pour test Limulus commercialisé par Mallenkrodt) et 200 µl du liquide à analyser contenant des endotoxines, liquide dont le pH est ajusté au préalable, si nécessaire, à une valeur comprise entre 6 et 7,5. Le mélange ainsi obtenu est alors traité suivant l'un ou l'autre des modes opératoires a) et b) suivants:

a) Le mélange est maintenu à 25°C (ou 37°C) pendant 30 minutes, puis on ajoute 0,2 ml d'une solution 25 mM de benzamidine dans le diméthylformamide pour arrêter la réaction d'hydrolyse, et on lit, dans l'heure qui suit, l'intensité de fluorescence à la longueur d'onde 460 nm, la longueur d'onde d'excitation étant 362 nm et la solution étant maintenu à température constante (par exemple 25°C) au moment de la lecture.

b) Le mélange est maintenu à 37°C et l'on enregistre en continu l'intensité de fluorescence à la longueur d'onde de 460 nm, la longueur d'onde d'excitation étant 362 nm.

Dans les deux cas, l'intensité de fluorescence obtenue est comparée à celle fournie, dans les mêmes conditions, par un mélange réactionnel contenant 400 µl de la solution du peptide de formule (IV), 200 µl de lysat d'amébocytes du limule et 200 µl d'une solution étalon d'endotoxine à 50 pg/ml. De la comparaison des résultats on déduit la teneur en endotoxine du liquide à analyser.

Il est possible d'éliminer l'interférence éventuelle sur le dosage des endotoxines de protéases contenues dans le liquide à analyser, en particulier dans le cas où ce liquide est un liquide physiologique, en procédant à l'essai suivant:

Le liquide à analyser, dont le pH est ajusté si nécessaire à 7,0, est porté à l'ébullition à 100°C pendant 10 minutes. Le précipité formé éventuellement est éliminé par centrifugation et la partie surnageante est utilisée pour le dosage des endotoxines suivant la technique décrite précédemment. Si le résultat obtenu est le même que celui obtenu en utilisant le liquide physiologique non traité à 100°C, le résultat obtenu avec le liquide non traité est correct. Si le résultat obtenu avec le liquide traité à 100°C est inférieur à celui obtenu avec le liquide non traité, seul sera retenu le résultat obtenu avec le liquide traité à 100°C.

Il est également possible de vérifier l'absence dans le liquide à analyser de protéases ou au contraire de produits inhibant l'hydrolyse du peptide de formule (IV) et de tenir compte dans le cas contraire, lors du dosage des endotoxines, de la présence de ces protéases ou produits dans l'échantillon à analyser en opérant comme suit:

On prépare les solutions A, B, C, D dont la composition est indiquée dans le tableau ci-après.

| | A | B | C | D |
|---|---|---|---|---|
| Lysat d'amébocytes de limule | 0 | 200 µl | 200 µl | 200 µl |
| Solution tampon phosphate 0,025 M-pH 7,4 | 300 µl | 100 µl | 0 | 100 µl |
| Solution de peptide de formule (IV) | 400 µl | 400 µl | 400 µl | 400 µl |
| Liquide à analyser | 100 µl | 0 | 100 µl | 100 µl |
| Solution étalon d'endotoxine à 50 pg/ml | 0 | 100 µl | 100 µl | 0 |

Les solutions A, B, C, D sont ensuite traitées suivant l'un ou l'autre des modes opératoires a) et b) précédemment définis. L'essai avec la solution A permet de déceler la présence éventuelle de protéases dans l'échantillon à analyser et de tenir compte de cette présence lors du dosage des endotoxines dans l'échantillon (essai avec la solution D). L'essai avec la solution C, qui contient un étalon interne, permet, par comparaison avec les essais relatifs aux solutions B et D, de déceler la présence éventuelle dans l'échantillon à analyser de produits inhibant l'hydrolyse du peptide de formule (IV) et de tenir compte de cette présence lors du dosage des endotoxines.

Exemple 4

Application du peptide de l'exemple 2 au dosage des endotoxines

On opère comme à l'exemple 3 en remplaçant au départ le peptide de formule (IV) par le peptide de formule (VII). La longueur d'onde d'excitation et la longueur d'onde à laquelle s'effectue la mesure de l'intensité de fluorescence, longueur d'onde qui est la longueur d'onde d'émission de l'amine libre, sont alors les suivantes:

| Peptide utilisé | longueur d'onde d'excitation | longueur d'onde à laquelle est mesurée l'intensité de fluorescence |
|---|---|---|
| Formule (VII) | 464 nm | 512 nm |

**Revendications**

1. Procédé de dosage fluorimétrique des endotoxines dans les liquides caractérisé en ce qu'il consiste à mettre en contact, à une température située dans l'intervalle 25°C-37°C, le liquide à analyser avec un lysat d'amébocytes du limule et une solution aqueuse d'un peptide de formule:

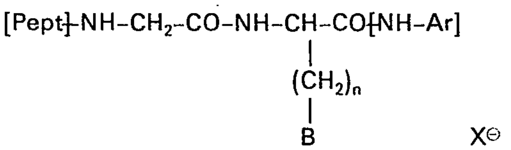

(I)

dans laquelle [Pept] désigne une chaîne peptidique hydrophobe, n est égal à 1, 2, 3, 4 ou 5, B est un radical

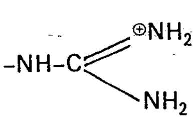

ou

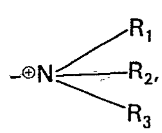

$R_1$, $R_2$ et $R_3$ désignant des atomes d'hydrogène ou des groupes méthyle, [NH-Ar] désigne le reste de l'amino-3 éthyl-9 carbazole ou de l'amino-7 nitro-4 oxa-2 benzodiazole-1,3 et $X^\ominus$ désigne un anion, à régler si nécessaire le pH du

milieu ainsi obtenu à une valeur comprise dans l'intervalle 6 à 7,5, et à doser par fluorimétrie l'amine hétéroaromatique $Ar\text{-}NH_2$ formée.

2. Procédé selon la revendication 1, caractérisé en ce que le liquide à analyser, le lysat d'amébocytes du limule et la solution aqueuse du peptide de formule (I) sont laissés en contact, après réglage éventuel du pH, pendant 10 minutes à 30 minutes, puis on ajoute au milieu un composé qui arrête l'hydrolyse du peptide de formule (I) sans gêner la fluorescence de l'amine formée, et on dose cette dernière en mesurant l'intensité de fluorescence à la longueur d'onde d'émission de l'amine libre.

3. Procédé selon la revendication 2, caractérisé en ce que le composé arrêtant l'hydrolyse du peptide de formule (I) sans gêner la fluorescence de l'amine formée est la benzamidine, l'ortho-amino benzamidine ou la para-amino benzamidine.

4. Procédé selon la revendication 1, caractérisé en ce que l'intensité de fluorescence à la longueur d'onde d'émission de l'amine libre est enregistrée en continu dès la mise en contact du liquide à analyser, du lysat d'amébocytes du limule et de la solution aqueuse du peptide de formule (I) et le réglage éventuel du pH.

5. Procédé selon l'une quelconque des revendications 1 à 4, caractérisé en ce que le peptide de formule (I) utilisé est tel que n est égal à 3,

B est le groupe 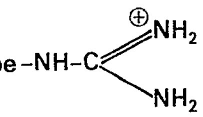

et la chaîne peptidique hydrophobe [Pept]- a 1 ou 2 chaînons acides aminés.

6. Procédé selon l'une quelconque des revendications 1 à 5, caractérisé en ce que le peptide de formule (I) utilisé répond à la formule:

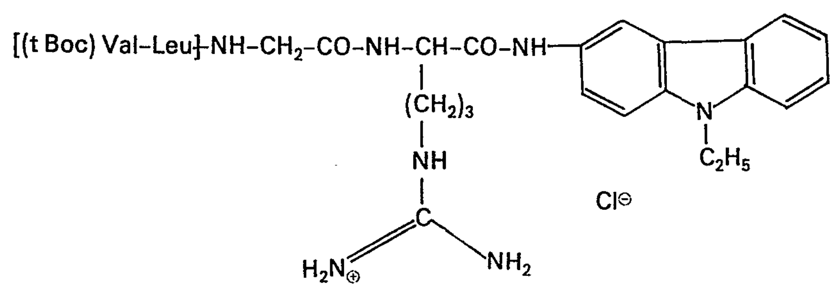

dans laquelle Leu est le chaînon leucine et (t Boc) Val le chaînon valine dans lequel le groupe amino $NH_2$ est substitué par un groupe tertiobutyloxycarbonyle.

7. Procédé selon l'une quelconque des revendications 1 à 5, caractérisé en ce que le peptide utilisé répond à la formule:

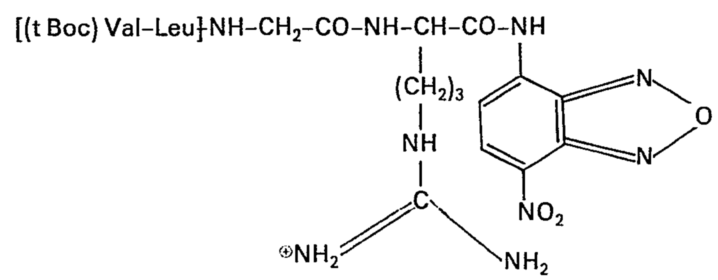

8. Peptides de formule:

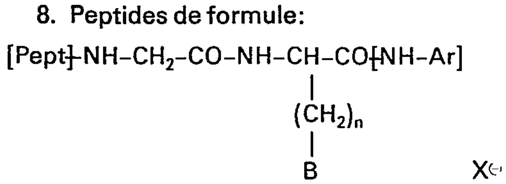

(I)

dans laquelle [Pept]- désigne une chaîne peptidique hydrophobe, n est égal à 1, 2, 3, 4 ou 5, B est un radical

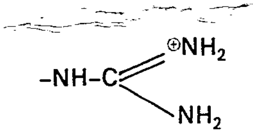

ou

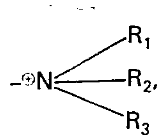

, $R_1$, $R_2$ et $R_3$

désignant des atomes d'hydrogène ou des groupes méthyle, -[NH-Ar] désigne le reste de l'amino-3 éthyl-9 carbazole ou de l'amino-7 nitro-4 oxa-2 benzodiazole-1,3 et $X^{\ominus}$ désigne un anion.

9. Peptides selon la revendication 8, caractérisé en ce que n est égal à 3 et B est le groupe

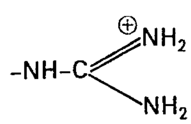

10. Peptides selon l'une quelconque des revendications 8 et 9, caractérisés en ce que la chaîne peptidique [Pept]- a 1 ou 2 chaînons acides aminés.

11. Peptides selon la revendication 10, caractérisés en ce que la chaîne peptidique hydrophobe [Pept]- est choisie parmi les chaînes [Val-Leu]-, [Ser (OBz)]-, [Ile-Glu ($\gamma$-$OCH_3$)]-, [Val]-, [Leu]-, [Val-Ser]-, chaînes dans lesquelles le groupe amino du chaînon acide aminé, lorsqu'il n'y a

qu'un seul chaînon, et le groupe amino du chaînon acide de gauche, lorsqu'il y a deux chaînons, sont non substitués ou substitués par un groupe tertiobutyloxycarbonyle, benzyloxycarbonyle, benzyle, tosyle ou acyle.

12. Peptide selon la revendication 11, caractérisé en ce qu'il a pour formule:

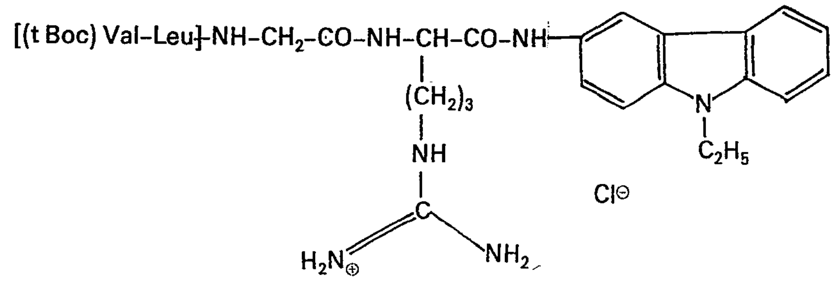

dans laquelle Leu est le chaînon leucine et (t Boc) Val le chaînon valine dans lequel le groupe amino $NH_2$ est substitué par un groupe tertiobutyloxycarbonyle.

13. Peptide selon la revendication 10, caractérisé par le fait qu'il a pour formule:

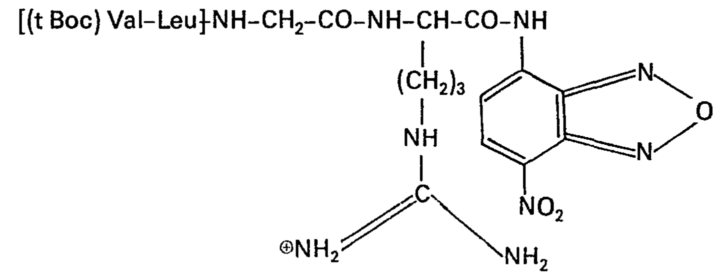

14. Procédé de préparation des peptides selon la revendication 8, caractérisé en ce que l'on fait agir, en présence d'un composé activateur du groupe COOH et d'hydroxy-1 benzotriazole, une amine choisie parmi l'amino-3 éthyl-9 carbazole et l'amino-7 nitro-4 oxa-2 benzodiazole-1,3 sur un peptide de formule générale:

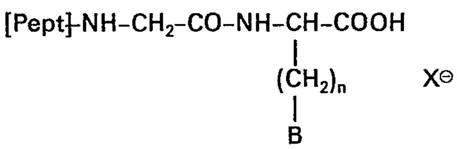 (II)

dans laquelle [Pept}, n, B et X⊖ ont les significations indiquées à la revendication 1.

**Patentansprüche**

1. Verfahren zur fluorimetrischen Bestimmung von Endotoxinen in Flüssigkeiten, dadurch gekennzeichnet, dass es im Messen bei einer Temperatur im Bereich von 25 bis 37°C von der zu analysierenden Flüssigkeit in Berührung mit einem Lysat von Amoebocyten von Limulus polyphemus und einer wässrigen Lösung eines Peptids der Formel

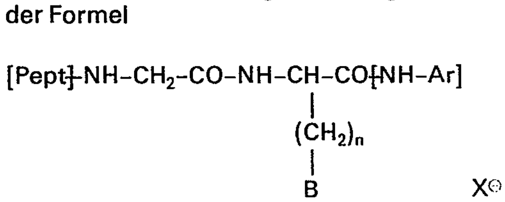 (I)

in der [Pept} eine hydrophobe Peptidkette bezeichnet, n gleich 1, 2, 3, 4 oder 5 ist, B ein Rest

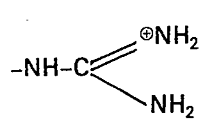 oder 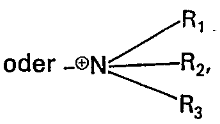 ist, wobei

$R_1$, $R_2$ und $R_3$ Wasserstoffatome oder Methylgruppen bezeichnen, {NH-Ar] den 3-Amino-9-ethyl-carbazolrest oder den 7-Amino-4-nitro-2-oxabenzodiazol-1,3-Rest bezeichnet und X⊖ ein Anion darstellt im Einstellen, falls erforderlich, des pH-Werts des so erhaltenen Mediums auf einen Wert im Bereich von 6 bis 7,5 und im fluorimetrischen Bestimmen des gebildeten heteroaromatischen Amins $Ar-NH_2$ besteht.

2. Verfahren nach Anspruch 1, dadurch gekennzeichnet, dass man die zu analysierende Flüssigkeit, das Lysat der Amoebocyten von Limulus und die wässrige Lösung des Peptids der Formel (I) nach eventueller pH-Wert-Einstellung 10 min bis 30 min in Berührung belässt, danach dem Medium eine Zubereitung zusetzt, bei der die Hydrolyse des Peptids der Formel (I) ohne Behinderung der Fluoreszenz des gebildeten Amins angehalten wird und dass man diese letztere Zubereitung zur Messung der Intensität der Fluoreszenz bei der Emissionswellenlänge des freien Amins bestimmt.

3. Verfahren nach Anspruch 2, dadurch gekennzeichnet, dass die Zubereitung, bei der die Hydrolyse des Peptids der Formel (I) ohne Behinderung der Aminofluoreszenz angehalten wurde, das Benzidin, o-Aminobenzamidin oder p-Aminobenzamidin ist.

4. Verfahren nach Anspruch 1, dadurch gekennzeichnet, dass man die Intensität der Fluoreszenz bei der Emissionswellenlänge des freien Amins kontinuierlich in Berührung mit der zu analysierenden Flüssigkeit, des Lysat der Amoebocyten von Limulus und der wässrigen Lösung des Peptids der Formel (I) und bei eventueller Regelung des pH-Werts aufzeichnet.

5. Verfahren nach den Ansprüchen 1 bis 4, dadurch gekennzeichnet, dass das verwendete Peptid der Formel (I) ein solches ist, bei dem n gleich 3 ist, B die Gruppe

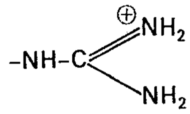

ist und die hydrophobe Peptidkette [Pept] 1 oder 2 saure aminierte Kettenglieder aufweist.

6. Verfahren nach den Ansprüchen 1 bis 5, dadurch gekennzeichnet, dass das verwendete Peptid der Formel (I) der folgenden Formel entspricht:

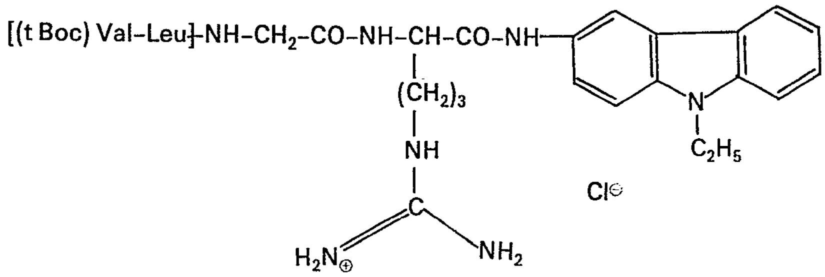

wobei in der Formel Leu ein Kettenglied des Leucins und (t Boc) Val das Valin-Kettenglied ist, bei welcher die Aminogruppe $NH_2$ durch eine t-Butyloxycarbonylgruppe substituiert ist.

7. Verfahren nach den Ansprüchen 1 bis 5, dadurch gekennzeichnet, dass das verwendete Peptid der Formel

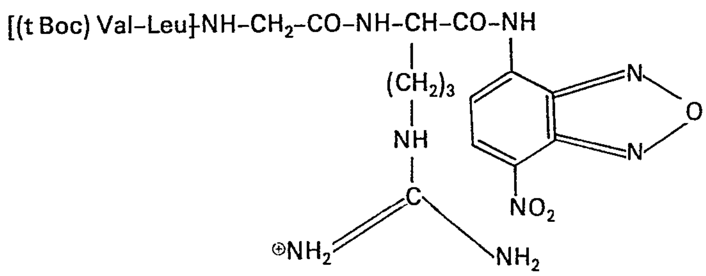

entspricht.

8. Peptide der Formel:

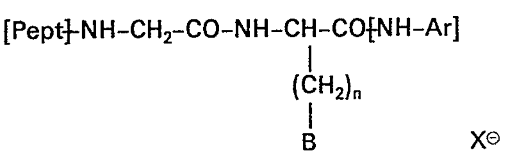 (I)

in welcher [Pept] eine hydrophobe Peptidkette darstellt, n gleich 1, 2, 3, 4 oder 5 und B ein Rest

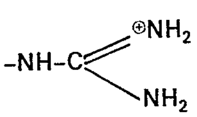 oder 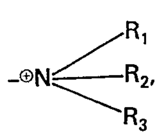 $R_1, R_2$ und $R_3$

Wasserstoffatome oder Methylgruppen bezeichnen, wobei [NH-AR] den 3-Amino-9-Methylcarbazolrest oder den 7-Amino-4-nitro-2-oxabenzodiazol-1,3-Rest und $X^{\ominus}$ ein Anion bezeichnen.

9. Peptide nach Anspruch 8, dadurch gekennzeichnet, dass in ihrer Formel n gleich 3 ist und B die Gruppe

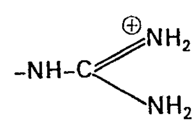

bedeutet.

10. Peptide nach Anspruch 8 oder 9, dadurch gekennzeichnet, dass die Peptidkette [Pept] 1 oder 2 saure, aminierte Kettenglieder aufweist.

11. Peptide nach Anspruch 10, dadurch gekennzeichnet, dass die hydrophobe Peptidkette [Pept] ausgewählt ist aus den Ketten [Val-Leu], [Ser (OBz)], [Ile-Glu (γ-$OCH_3$)], [Val], [Leu], [Val-Ser], wobei in diesen Ketten die Aminogruppe des sauren aminierten Kettenglieds einzeln oder nicht stehen und die Aminogruppe des linken sauren Kettenglieds oder beide Kettenglieder unsubstituiert oder durch eine tert.-Butyloxycarbonyl-, Benzyloxycarbonyl-, Benzyl-, Tosyl- oder Acylgruppe substituiert sind.

12. Peptid nach Anspruch 11, dadurch gekennzeichnet, dass es der folgenden Formel entspricht:

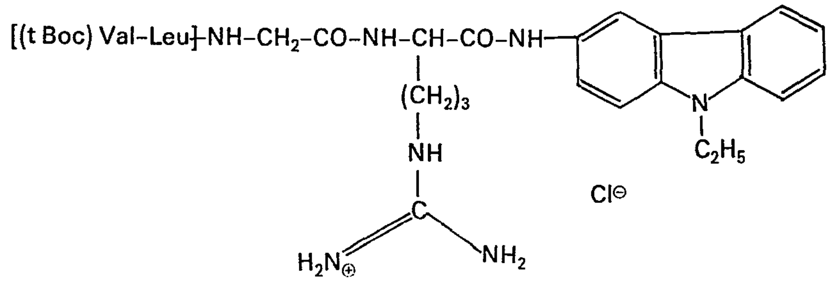

wobei Leu ein Leucin-Kettenglied und (t Boc) Val das Valinkettenglied ist, wobei die Aminogruppe $NH_2$ durch eine tert.-Butyloxycarbonylgruppe substituiert ist.

13. Peptid nach Anspruch 10, dadurch gekennzeichnet, dass es der folgenden Formel entspricht:

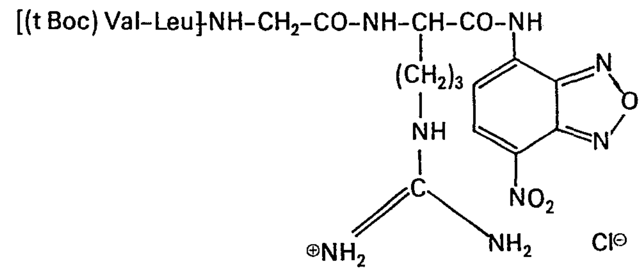

14. Verfahren zur Herstellung des Peptids nach Anspruch 8, dadurch gekennzeichnet, dass man eine Behandlung in Gegenwart einer Aktivatorzubereitung aus der Gruppe COOH und 1-Hydroxybenzotriazol mit einem Amin, ausgewählt aus 3-Amino-9-ethylcarbazol und 7-Amino-4-nitro-2-oxabenzodiazol-1,3 auf einem Peptid der allgemeinen Formel

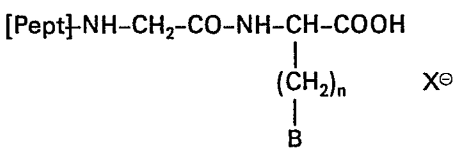 (II)

in der [Pept}, n, B und X⊖ die in Anspruch 1 genannte Bedeutung haben, vornimmt.

## Claims

1. Process for fluorimetric assay of endotoxins in liquids, characterised in that it consists in bringing the liquid to be analysed into contact, at a temperature in the range 25°C–37°C, with a lysate of king crab amoebocytes and an aqueous solution of a peptide of formula:

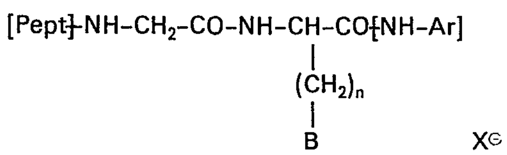 (I)

in which [Pept} denotes a hydrophobic peptide chain, n equals 1, 2, 3, 4 or 5, B is a

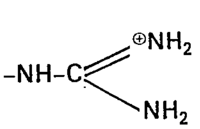 or 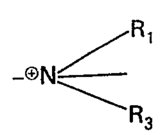

radical, $R_1$, $R_2$ and $R_3$ denoting hydrogen atoms or methyl groups, {NH–Ar] denotes a 3-amino-9-ethylcarbazole or 7-amino-4-nitro-2-oxa-1,3-benzodiazole residue, and X⊖ denotes an anion, in adjusting if necessary the pH of the medium thereby obtained to a value in the range 6 to 7.5, and in assaying by fluorimetry the heterocyclic aromatic amine $Ar–NH_2$ formed.

2. Process according to Claim 1, characterised in that the liquid to be analysed, the lysate of king crab amoebocytes and the aqueous solution of the peptide of formula (I) are left in contact, after adjustment of pH if appropriate, for 10 minutes to 30 minutes, the compound which stops the hydrolysis of the peptide of formula (I) without interfering with the fluorescence of the amine formed is then added to the medium, and the amine formed is assayed by measuring the fluorescence intensity at the wavelength of emission of the free amine.

3. Process according to Claim 2, characterised in that the compound which stops the hydrolysis of the peptide of formula (I) without interfering with the fluorescence of the amine formed is benzamidine, ortho-aminobenzamidine or para-aminobenzamidine.

4. Process according to Claim 1, characterised in that the fluorescence intensity at the wavelength of emission of the free amine is recorded continuously from the time at which the liquid to be analysed, the lysate of king crab amoebocytes and the aqueous solution of the peptide of formula (I) are brought into contact and the pH is adjusted if appropriate.

5. Process according to any one of Claims 1 to 4, characterised in that the peptide of formula (I) used is such that n equals 3, B is a 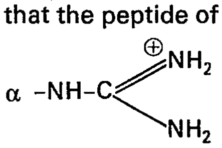

group and the hydrophobic peptide chain [Pept]- has 1 or 2 amino acid units.

6. Process according to any one of Claims 1 to 5, characterised in that the peptide of formula (I) used corresponds to the formula:

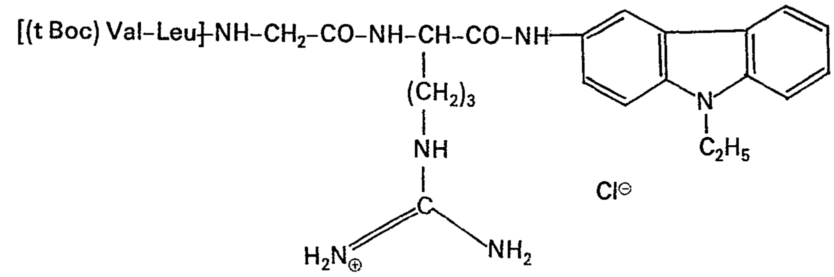

in which Leu is a leucine unit and (t Boc) Val a valine unit in which the $NH_2$ amino group is substituted with a tert-butyloxycarbonyl group.

7. Process according to any one of Claims 1 to 5, characterised in that the peptide used corresponds to the formula:

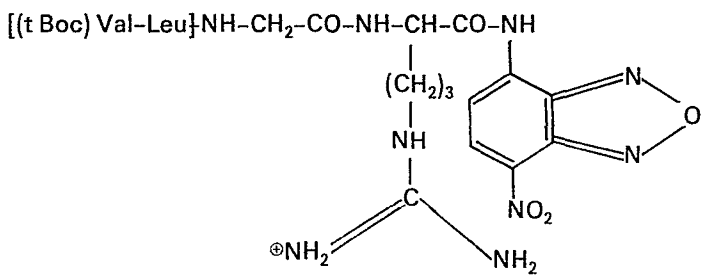

8. Peptides of formula:

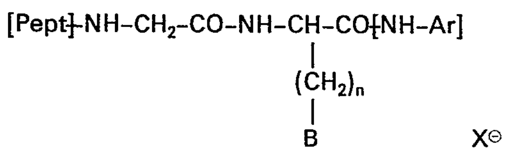

(I)

in which [Pept]- denotes a hydrophobic peptide chain, n equals 1, 2, 3, 4 or 5, B is a

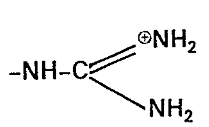

or

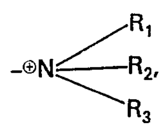

radical, $R_1$, $R_2$ and $R_3$ denoting hydrogen methyl groups, -[NH-Ar] denotes a 3-amino-9-ethylcarbazole or 7-amino-4-nitro-2-oxa-1,3-benzodiazole residue and $X^{\ominus}$ denotes an anion.

9. Peptides according to Claim 8, characterised in that n equals 3 and B is a

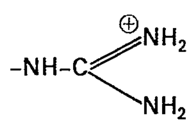

group.

10. Peptides according to either one of Claims 8 and 9, characterised in that the peptide chain [Pept]- has 1 or 2 amino acid units.

11. Peptides according to Claim 10, characterised in that the hydrophobic peptide chain [Pept]- is chosen from the chains [Val-Leu]-, [Ser(OBz)]-, [Ile-Glu(γ-$OCH_3$)]-, [Val]-, [Leu]-, [Val-Ser]-, in which chains the amino group of the amino acid unit, when there is only a single unit, and the amino group of the acid unit to the left, when there are two units, are unsubstituted or are substituted with a tert-butyloxycarbonyl, benzyloxycarbonyl, benzyl, tosyl or acyl group.

12. Peptide according to Claim 11, characterised in that it has the formula:

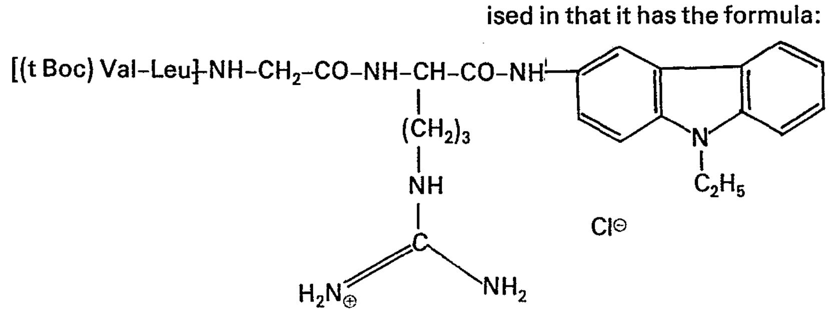

in which Leu is a leucine unit and (t Boc) Val a valine unit in which the $NH_2$ amino group is substituted with a tert-butyloxycarbonyl group.

13. Peptide according to Claim 10, characterised in that it has the formula:

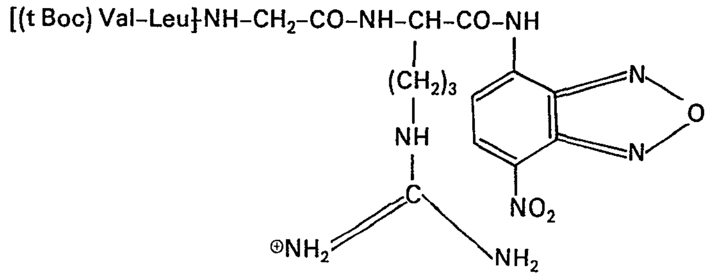

14. Process for preparing the peptides according to Claim 8, characterised in that an amine chosen from 3-amino-9-ethylcarbazole and 7-amino-4-nitro-2-oxa-1,3-benzodiazole is reacted, in the presence of a compound which activates the COOH group and of 1-hydroxybenzotriazole, with a peptide of general formula:

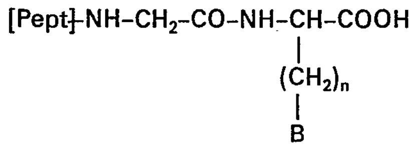 $X^{\ominus}$ (II)

in which [Pept]-, n, B and $X^{\ominus}$ have the meanings given in Claim 1.